# EUROPÄISCHE PATENTSCHRIFT

(11) **EP 1 694 257 B1**
(45) Veröffentlichungstag und Bekanntmachung des Hinweises auf die Patenterteilung: **12.08.2009**
(21) Anmeldenummer: 04820061.2
(22) Anmeldetag: 10.12.2004
(51) Int. Cl.: A61F 2/44

(54) **LÄNGENVERSTELLBARES WIRBELSÄULEN-IMPLANTAT**
LENGTH-ADJUSTABLE IMPLANT FOR THE VERTEBRAL COLUMN
IMPLANT POUR COLONNE VERTEBRALE POUVANT ETRE AJUSTE EN LONGUEUR

(30) Priorität: 11.12.2003 DE 10357926
(43) Veröffentlichungstag der Anmeldung: 30.08.2006
(73) Patentinhaber: TPL - Kilian Kraus, 97440 Werneck (DE)
(72) Erfinder: KRAUS, Kilian, 97440 Werneck (DE)
(74) Vertreter: Mörtel & Höfner
(86) Internationale Anmeldenummer: PCT/EP2004/014060
(87) Internationale Veröffentlichungsnummer: WO 2005/055887

(56) Entgegenhaltungen:
- DE-C1- 10 127 924
- US-A1- 2003 045 877

## Beschreibung

Die Erfindung betrifft ein längenverstellbares Implantat zum Einsetzen zwischen Wirbelkörpern. Ein dafür geeignetes Handhabungswerkzeug wird ebenfalls offenbart. Beispielsweise aus DE 196 22 827 A1 und US 2003/0045877 A1 bekannte Implantate weisen ein erstes und ein zweites Hülsenteil auf, wobei die beiden Hülsenteile bei koaxialer Ausrichtung drehfest und axial beweglich verbunden sind und das erste Hülsenteil ein Außengewinde trägt. Zur axialen Verstellung der beiden Hülsen gegeneinander ist eine Mutter vorgesehen, die drehbar am zweiten Hülsenteil fixiert ist und mit ihrem Gewinde in das Außengewinde des ersten Hülsenteils eingreift. Durch Drehen der Mutter werden die beiden Hülsenteile relativ zueinander axial bewegt. Zur Drehbetätigung der Mutter wird ein stabförmiges Handhabungswerkzeug in eine Ausnehmung am Außenumfang der Mutter eingesteckt. Durch eine Schwenkbewegung des Werkzeugs in einer quer zur Längsachse des Implantats verlaufenden Ebene wird die Mutter um ein dem Schwenkwinkel des Werkzeugs entsprechendes Stück weiter gedreht. Danach muss das Werkzeug wieder aus der Mutter herausgezogen und der geschilderte Vorgang so lange wiederholt werden, bis das Implantat eine den Raum zwischen zwei Wirbelkörpern überbrückende Länge aufweist. Während der Drehbetätigung der Mutter darf sich das zweite Hülsenteil nicht mitdrehen, so dass dieses im Regelfall mit einem weiteren Werkzeug festgehalten werden muss. Die Längenverstellung bei den bekannten Implantaten erfordert somit einen erhöhten Zeit- und Gerätetechnischen Aufwand. Außerdem ist eine relativ große Operationsöffnung erforderlich, um die genannten Manipulationen, insbesondere die Schwenkung des Handhabungswerkzeugs behinderungsfrei vornehmen zu können. Nachteil-bei den bekannten Implantaten ist weiterhin, das sowohl durch die Schwenkbewegung des Werkzeuges als auch durch die Drehbewegung der Mutter umliegendes Gewebe verletzt werden kann. Aus DE 101 27 924 A1 ist ein Implantat bekannt, das ähnlich aufgebaut ist wie die weiter oben beschriebenen. Die Drehbetätigung zur Höhenverstellung erfolgt über eine Getriebeeinheit. Die Getriebeeinheit umfasst eine seitlich am Implantat angesetzte Schnecke, die mit Hilfe eines Handhabungswerkzeuges betätigbar ist.

Davon ausgehend ist es Aufgabe der Erfindung, ein Implantat mit verbesserter Handhabbarkeit und vergrößertem Innenraum zur Aufnahme von Knochenmaterial vorzuschlagen.

Diese Aufgabe wird hinsichtlich eines Implantats durch Anspruch 1 gelöst. Gemäß Anspruch 1 ist vorgesehen, dass das erste Hülsenteil ein Innengewinde trägt und dass eine ein Außengewinde tragende Mutter in dem von den Hülsenteilen umgrenzten Innenraum des Implantats koaxial angeordnet sowie drehbar und axialfest am zweiten Hülsenteil fixiert ist. Die Mutter greift mit ihrem Außengewinde in das Innengewinde des ersten Hülsenteils ein und trägt einen koaxial zu den Hülsenteilen verlaufenden Zahnring angeordnet, welcher über eine Zugangsöffnung im zweiten Hülsenteil zugänglich ist.

Bei dieser Ausgestaltung ist zunächst von Vorteil, das zur Drehbetätigung der Mutter mit einem Handhabungswerkzeug keine Schwenkbewegungen ausgeführt werden müssen. Vielmehr ist es nun möglich, das Handhabungswerkzeug während des gesamten Vorganges der Längenverstellung des Implantates in ein und derselben Stellung bzw. Ausrichtung zu halten, was die Operation wesentlich vereinfacht und Operationszeit spart. Außerdem ist nur eine relativ kleine Operationsoffnung erforderlich. Der Antrieb kann auf einfache Weise z.B. mit einem am einen Ende eines Handhabungswerkzeugs angeordneten Antriebszahnrad erfolgen. Ein weiterer wesentlicher Vorteil des vorgeschlagenen Implantats besteht darin, dass die Mutter in dessen Innenraum angeordnet ist, so dass bei deren Drehung eine Verletzungsgefahr für das umliegende Gewebe ausgeschlossen ist. Dies trifft auch für ein während der Längenverstellung mit dem Zahnring kämmendes Antriebszahnrad eines Handhabungswerkzeugs zu. Eine Verletzung umliegenden Gewebes durch die zwei sich relativ zueinander axial bewegenden Hülsenteile kann aufgrund deren glatter Außenoberfläche praktisch ausgeschlossen werden.

Die Zugangsöffnung gewährleistet nicht nur einen behinderungsfreien Zugang zum Zahnring, sondern dient auch zur Fixierung eines Handhabungswerkzeuges am Implantat: Das entsprechende Ende des Werkzeugs ist dabei so ausgebildet, dass es in der Zugangsöffnung fixierbar, z.B. darin passgenau einsteckbar oder darin einschraubbar ist. Das Implantat ist dann sicher mit dem Handhabungswerkzeug verbunden, so dass dieses nicht nur zum Drehbetätigen der Mutter, sondern auch zum sicheren und positionsgenauen Einsetzen des Implantats in die Wirbelsäule dienen kann. Weiterhin ist durch diese Ausgestaltung eine Drehfixierung des zweiten Hülsenteils während der Längenverstellung des Implantats gewährleistet. Im Gegensatz zu herkömmlichen Implantaten ist somit nur ein einziges, zum Antrieb der Mutter, zum Einsetzen des Implantats und zur Drehfixierung des zweiten Hülsenteils dienendes Werkzeug erforderlich.

Besonders vorteilhaft ist, dass die beiden Hülsenteile axial verlaufend, in ihren einander zugewandten Enden ausmündende Fenster aufweisen, wobei die zwischen zwei benachbarten Fenstern angeordneten Umfangsabschnitte in den Fenstern des jeweils anderen Hülsenteils axial verschiebbar einliegen. Gegenüber einer Anordnung, bei der zwei Hülsenteile mit unterschiedlichen Durchmessern konzentrisch ineinander greifen, hat dies zunächst den Vorteil einer Material- und Gewichtseinsparung. Außerdem steht ein größerer Innenraum zur Verfügung, der mit Knochenmaterial oder dergleichen aufgefüllt werden kann. Durch das kammartige Ineinandergreifen der beiden Hülsenteile ist weiterhin eine gegenseitige Drehfixierung gewährleistet.

Bei einer bevorzugten Ausgestaltung ist der Zahnring nach Art eines Kronenrades ausgebildet. Dadurch kann das Antriebsritzel des Handhabungswerkzeugs mit dem Zahnring nach Art eines Kronenradgetriebes zusammenwirken. Daraus ergibt sich der Vorteil, dass die Drehachse des Antriebszahnrades eines Handhabungswerkzeugs etwa radial zur Mittelängsachse des Implantats und dementsprechend in Längsrichtung eines zweckmäßigerweise stabförmigen Handhabungswerkzeugs verläuft. Dieses kann daher relativ einfach gestaltet sein indem es nämlich nur eine zentral angeordnete Drehachse aufweist, an deren zur Drehbetätigung der Mutter dienendem Ende ein Antriebszahnrad fixiert ist.

Der Eingriff der Mutter in das Innengewinde des ersten Hülsenteile ist vorzugsweise dadurch gewährleistet, dass diese so am zweiten Hülsenteil fixiert ist, dass sich ihr Außengewinde in Axialrichtung gesehen außerhalb des zweiten Hülsenteils befindet. Denkbar ist auch eine Anordnung der Mutter, bei der sich ihr Gewinde - wiederum in Axialrichtung gesehen - innerhalb des zweiten Hülsenteils befindet. Das Innengewinde des ersten Hülsenteils müsste dann, damit es in das Muttergewinde eingreifen kann, über die Innenfläche des zweiten Hülsenteils hinausstehen, indem es z.B. eine größere Wandstärke aufweist als das zweite Hülsenteil. Bei der bevorzugten Anordnung der Mutter ist eine solche Maßnahme dagegen nicht erforderlich.

Vorzugsweise ist der Zahnring auf einer vom Außengewinde weg weisenden Fläche der Mutter angeordnet, was in fertigungstechnischer Sicht vorteilhaft ist. Außerdem kann Länge der Mutter gering gehalten werden. Wäre der Zahnring auf einer dem Außengewinde zugewandten Fläche angeordnet, müsste zwischen dem Außengewinde und dem Zahnring ein größerer, nämlich mindestens ein dem Durchmesser eines in den Zahnring eingreifenden Antriebszahnrads entsprechender Axialabstand vorhanden sein.

Zur axialen Fixierung der Mutter ist an den Innenseiten der Umfangsabschnitte des zweiten Hülsenteils jeweils ein radial nach innen vorstehender Vorsprung angeordnet, der in eine Umfangsnut der Mutter eingreift. Auf diese Weise ist eine sichere Axialfixierung der Mutter insbesondere auch im Belastungsfalle gewährleistet. Zur Monatage der Mutter am ersten Hülsenteil wird diese auf dieses aufgesteckt. Das Hülsenteil besteht zwar aus einem festen Material insbesondere aus einem Metall. Die in Umfangsrichtung voneinander getrennten Umfangsabschnitte weisen jedoch eine solche Elastizität auf, dass sie beim Aufstecken der Mutter nach innen abgelenkt werden und dann wieder in ihre Ausgangsstellung zurückfedern, wobei die Vorsprünge in die Umfangsnut der Mutter einschnappen. Die Positionierung der Vorsprünge nahe dem Freiende der Umfangsabschnitte hat den Vorteil, dass die Mutter relativ kurz gehalten werden kann, ohne dass dadurch der maximale Verstellweg des Implantats verringert würde. Außerdem ist mit dieser Ausgestaltung eine Material- und Gewichtseinsparung sowie eine Vergrößerung des Innenraumes des Implantats verbunden.

Zur Vergrößerung der Stützfläche des Implantats ist eine radial verbreitete Stützplatte vorgesehen, die bei einer bevorzugten Ausführungsform ein separates, lösbar an einem Hülsenteil fixiertes Teil ist. Es kann dann in jedem Einzelfall die geeignete Stützplatte, beispielsweise eine mit einer schräg zur Mittellängsachse des Implantats verlaufenden Planebene eingesetzt werden. Denkbar ist auch eine Stützplatte, die schwenkbar an einem Hülsenteil fixiert ist. Auch eine Drehfixierung kann vorteilhaft sein, beispielsweise um zu verhindern, dass ein zwischen zwei Wirbelkörper eingesetztes Implantat bei der Drehbetätigung des Zahnrings seine relative Drehstellung zu den Wirbelkörpern bzw. der Wirbelsäule beibehält. Vorzugsweise wird die Drehfixierung dadurch bewerkstelligt, dass in einer Stirnseite eines Hülsenteils mehrere in Umfangsrichtung beabstandete, sich in die Innenseite des Hülsenteils öffnende Ausnehmungen vorhanden sind, in die komplementär geformte Vorsprünge der Stützplatte eingreifen.

Ein Handhabungswerkzeug für ein Implantat der vorbeschriebenen Art weist einen Handgriff und eine diesen durchsetzenden Welle auf, die an ihrem eine Ende ein Zahnrad und an ihrem anderen Ende einen Drehgriff trägt. Mit einem solchen Werkzeug kann ein Implantat auf einfache und sichere Weise manipuliert werden. Dadurch, dass der sich zwischen dem Handgriff und dem Zahnrad erstreckende Abschnitt der Welle innerhalb eines Hüllrohrs verläuft, kann diese nicht mit Gewebe des Operationsbereichs in Berührung kommen, was zu unerwünschten Komplikationen führen könnte. Darüber hinaus dient das Hüllrohr auch zur Fixierung des Handhabungswerkzeugs am Implantat. Dazu ist sein vorderer, dem Zahnrad zugewandter Endabschnitt so ausgebildet ist, dass er in der Zugangsöffnung des Implantats fixierbar ist, z.B. in diese passgenau eingesteckt oder in diese eingeschraubt wird.

Die Erfindung wird nun anhand der beigefügten Zeichnungen näher erläutert. Es zeigen:
- Fig. 1: eine schematisierte Schnittdarstellung eines ersten Ausführungsbeispieles eines längenverstellbaren Implan- tats, welches als Hauptbestandteile ein erstes und ein zweites Hülsenteil sowie eine Mutter umfasst,
- Fig. 2: ein zweites Ausführungsbeispiel eines Implantats in einer perspektivischen Darstellung, das ebenfalls die genannten Hauptbestandteile umfasst,
- Fig. 3: das im Implantat von Fig. 2 aus einem anderen Blickwin- kel gesehen,
- Fig. 4: das zweite Hülsenteil des Implantats von Fig. 2 in einer perspektivischen Darstellung,
- Fig. 5: einen Querschnitt entsprechend Linie V-V in Fig. 3,
- Fig. 6: eine Mutter in perspektivischer Darstellung
- Fig. 7, 7A: eine an einem Implantat fixierbare Stützplatte,
- Fig. 8: ein Handhabungswerkzeug zur Manipulierung eines Implan- tats,
- Fig. 9: einen vergrößerten Ausschnitt aus Fig.8,
- Fig. 10: ein Ausführungsbeispiel mit einem Verstärkungsring in einer aufgebrochenen Seitenansicht,
- Fig. 11: die perspektivische Ansicht eines gegenüber Fig. 2 abgewandelten Implantats,
- Fig. 12, 12A: eine an dem Implantat von Fig. 12 fixierbare Stützplatte.

Zur Beschreibung der in den Abbildungen gezeigten Implantate, die jeweils ein erstes Hülsenteil 1, 101 ein zweites Hülsenteil 2, 102 und eine Mutter 3, 103 aufweisen, wird aus Vereinfachungsgründen auf ein vertikal ausgerichtetes Implantat Bezug genommen, das sich mit seinem ersten Hülsenteil auf einer Unterlage abstützt.

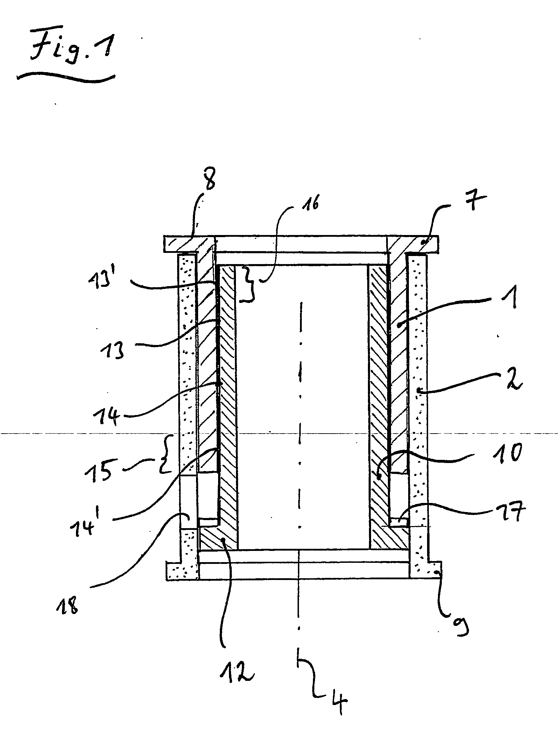

Bei dem Ausführungsbeispiel nach Fig.1 sind die beiden Hülsenteile 1,2 koaxial zueinander ausgerichtet und derart ineinander gesteckt, dass sie teleskopartig gegeneinander in Richtung ihrer Mittellängsachse 4 verschiebbar sind. Der Innendurchmesser des Hülsenteils 2 ist geringfügig, größer als der Außendurchmesser des Hülsenteils 1, so dass die Hülsenteile bei einer relativen axialen Verschiebung im Sinne einer Gleitpaarung zusammenwirken. Ein aus dem Hülsenteil 2 hervorstehender Endabschnitt des Hülsenteiles 1 trägt einen radial nach außen abstehenden Flansch 7, dessen dem Hülsenteil 2 abgewandte Oberseite 8 eine Stützfläche bildet. Der Flansch 7 dient darüber hinaus als Endanschlag für die Einfahrbewegung des Hülsenteils 1 in das Hülsenteil 2. An der dem Flansch 7 abgewandten, unteren Stirnseite des Hülsenteiles 2 ist ebenfalls ein dem gleichen Zweck dienender Flansch 9 angeordnet.

Die beiden Hülsenteile 1, 2 sind axial verschiebbar jedoch drehfixiert ineinander gelagert. Die Drehfixierung kann etwa dadurch bewerkstelligt sein, dass sich in Richtung der Mittellängsachse 4 erstreckende Rippen an der Außenseite des Hülsenteils 1 in entsprechenden Nuten in der Innenseite des Hülsenteiles 2 eingreifen. Zur axialen Verschiebung der beiden Hülsenteile 1, 2 ist eine Mutter 10 vorgesehen. Diese ist in Form eines Rohrabschnitts ausgebildet, der an seinem unteren Ende einen sich radial nach außen erstreckenden Flansch 12 trägt, der an der Innenfläche des zweiten Hülsenteils 2 anliegt. Die Mutter 10 greift mit einem Außengewinde 13 in ein Innengewinde 14 des ersten Hülsenteils 1 ein. Das Innengewinde 14 erstreckt sich nahezu über.die gesamte Länge des Hülsenteiles 1. Das Außengewinde 13 der Mutter 10 weist eine entsprechende Länge auf. Es wäre natürlich auch ausreichend, wenn das Hülsenteil 1 an seinem unteren Ende nur einen kurzen, sich etwa über den Bereich 15 erstreckenden Innengewindeabschnitt 14' aufweisen würde. Denkbar wäre auch ein kurzer Außengewindeabschnitt 13' am oberen Ende der Mutter 10, der sich etwa über einen Bereich 16 erstrecken könnte. Das Innengewinde 14 des Hülsenteils 1 wäre dann je nach dem gewünschten Ausmaß der Höhenverstellung des Implantats entsprechend länger. Auf der Oberseite des Flansches 12 ist ein Zahnring 17 angeordnet, der zur Drehbetätigung der Mutter 10 dient. Flansch 12 und Zahnring 17 entsprechen dem Kronenrad eines Kronenradgetriebes. Im Bereich des Zahnringes 17 ist das zweite Hülsenteil 2 von einer Zugangsöffnung 18 radial durchsetzt. Durch diese kann ein mit dem Zahnring 17 zusammenwirkendes Handhabungswerkzeug, welches weiter unten noch näher beschrieben wird, eingeführt werden. Durch eine Drehung der Mutter 10 wird das Hülsenteil 1 aus dem Hülsenteil 2 heraus und, falls erforderlich, auch wieder hinein bewegt. Die Auswärtsbewegung ist zweckmäßiger Weise durch einen Anschlag (nicht dargestellt) begrenzt. In den Hülsenteilen 1, 2 sowie in der Mutter 10 können weitere Radialöffnungen (nicht dargestellt) vorhanden sein, um den Innenraum des in der Wirbelsäule platzierten Implantats mit Knochenersatzmaterial o. dgl. zu füllen.

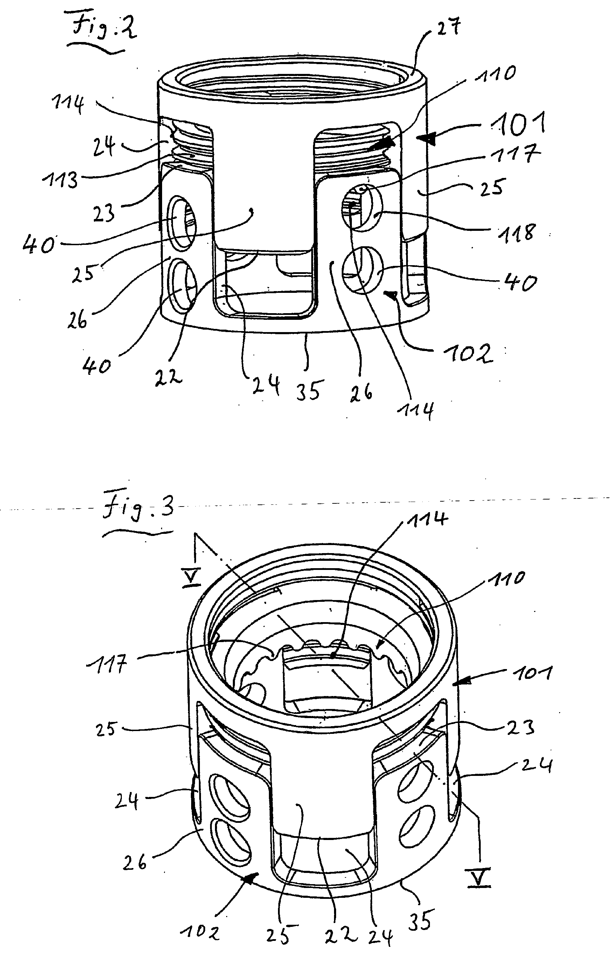

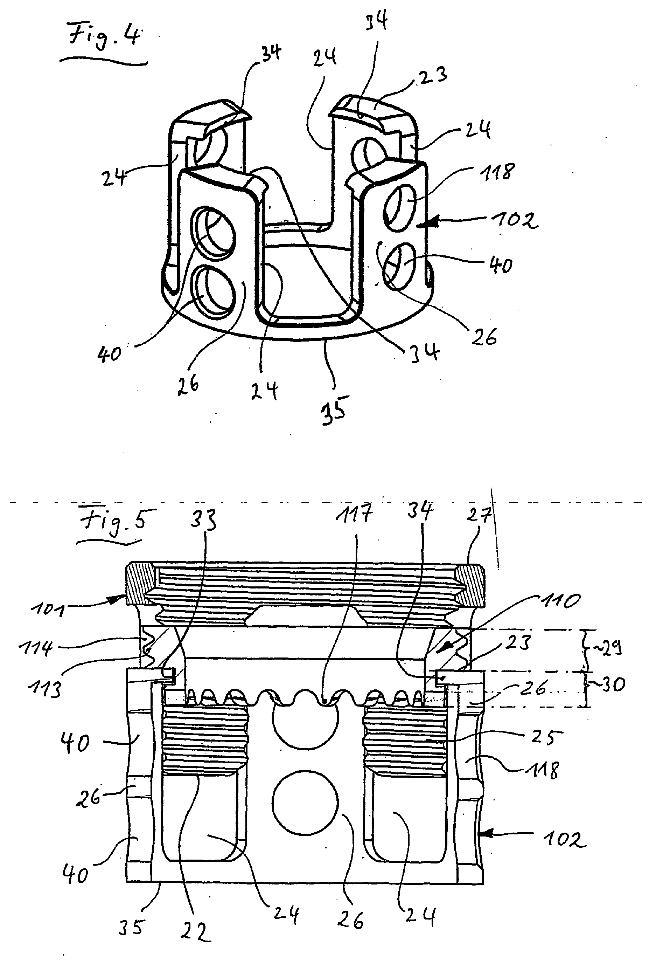

Ein weiteres Ausführungsbeispiel eines längenverstellbaren Implantats ist in Fig. 2 bis 6 dargestellt. Die beiden Hülsenteile 101 und 102 weisen axial verlaufende, in ihren im montierten Zustand in ihre einander zugewandten Stirnseiten 22, 23 ausmündende Fenster 24 auf. Die jeweils zwei benachbarte Fenster 24 voneinander trennenden Umfangsabschnitte 25, 26 liegen in den Fenstern 24 des jeweils anderen Hülsenteils 101, 102 axial verschiebbar ein. Das Spiel zwischen den Umfangsabschnitten 25, 26 ist dabei so bemessen, das ein wackelfreier Sitz der beiden Hülsenteile 101, 102 ineinander und dennoch eine leichte Verschiebbarkeit gewährleistet ist.

Zur Längenverstellung bzw. zum Ein- und Ausfahren des Hülsenteils 1 dient die Mutter 110, welche koaxial innerhalb des ersten Hülsenteils 1 angeordnet ist und mit ihrem Außengewinde 113 in das Innengewinde 114 des ersten Hülsenteils 1 eingreift. Das Innengewinde 114 des ersten Hülsenteils 101 erstreckt sich etwa von der unteren Stirnseite 22 des Hülsenteils 1 bis zu dessen oberer Stirnseite 27.

Die Mutter 110 ist hülsenförmig ausgebildet und weist einen das Außengewinde 113 tragenden Längsabschnitt 29 und einen Gewindefreien Längsabschnitt 30 auf. Der Längsabschnitt 29 bzw. das Außengewinde 114 ist oberhalb des zweiten Hülsenteils 102 angeordnet.

Die Stirnseite des Längsabschnitts 29, die im Montagzustand zum Hülsenteil 2 bzw. nach unten weist, ist als Zahnring 117 ausgebildet. Der Außendurchmesser des Zahnrings 117 ist kleiner als der Außendurchmesser des Außengewindes 113 und kleiner als der Innendurchmesser des Hülsenteils 102. Der Außendurchmesser des Gewindes 114 der Mutter 110 ist hingegen größer als der Innendurchmesser des Hülsenteils 102 und so bemessen, dass das Gewinde 114 mit dem Innengewinde 113 des ersten Hülsenteils 101 in Eingriff steht.

Zwischen dem Längsabschnitt 29 und dem Längsabschnitt 30 befindet sich ein eine Ringnut 33 bildender Einstich. Zur axialen Fixierung der Mutter 110 am Hülsenteil 102 weist dieses radial nach innen abstehende Vorsprünge 34 auf, die in die Ringnut 33 der Mutter 110 eingreifen. Die Vorsprünge 34 sind am Freiende der Umfangsabschnitte 26 so angeordnet, dass ihre Oberseite mit der oberen Stirnseite 23 der Umfangsabschnitte 26 fluchtet. Die Hauptbestandteile des Implantats, also Hülsenteil 101, Hülsenteil 102 und Mutter 110 sind vorzugsweise aus Metall gefertigt. Die Umfangsabschnitte 26 lassen sich dennoch bei der Montage der Mutter 110 geringfügig radial nach außen biegen, so dass die Mutter 110 mit ihrem Längsabschnitt 30 in das Hülsenteil 2 eingesetzt werden kann, wobei die Vorsprünge 34 in die Ringnut 33 einschnappen.

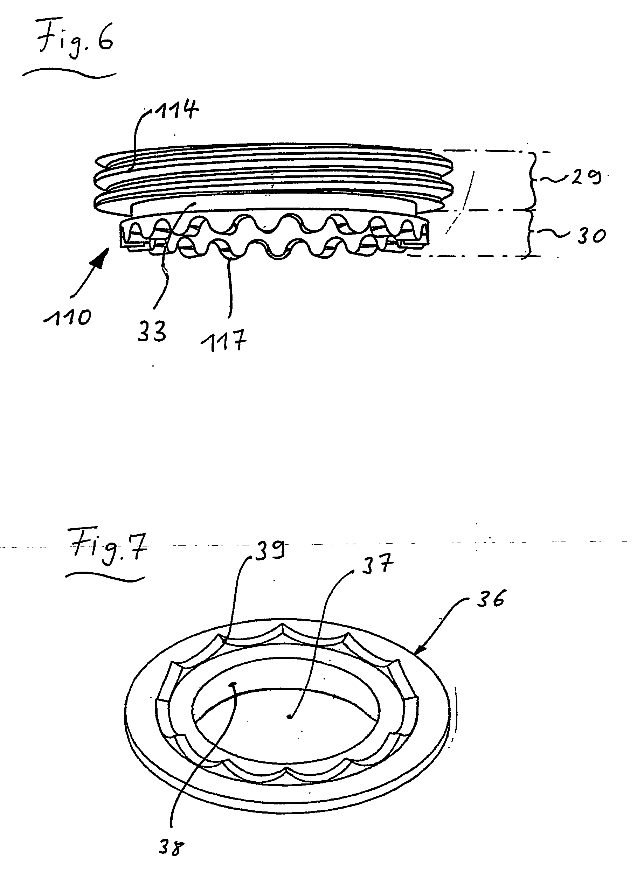

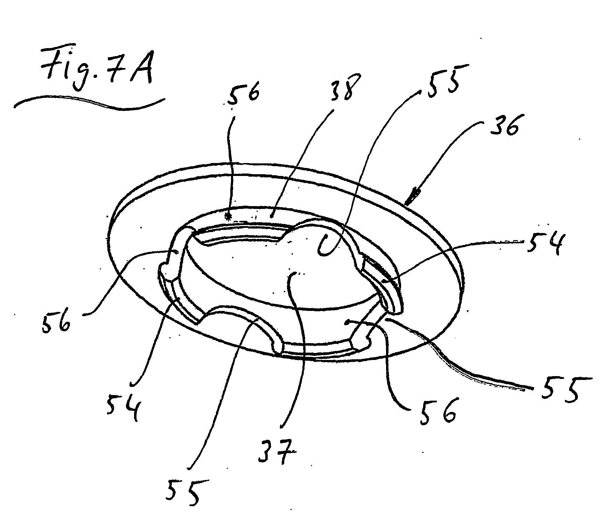

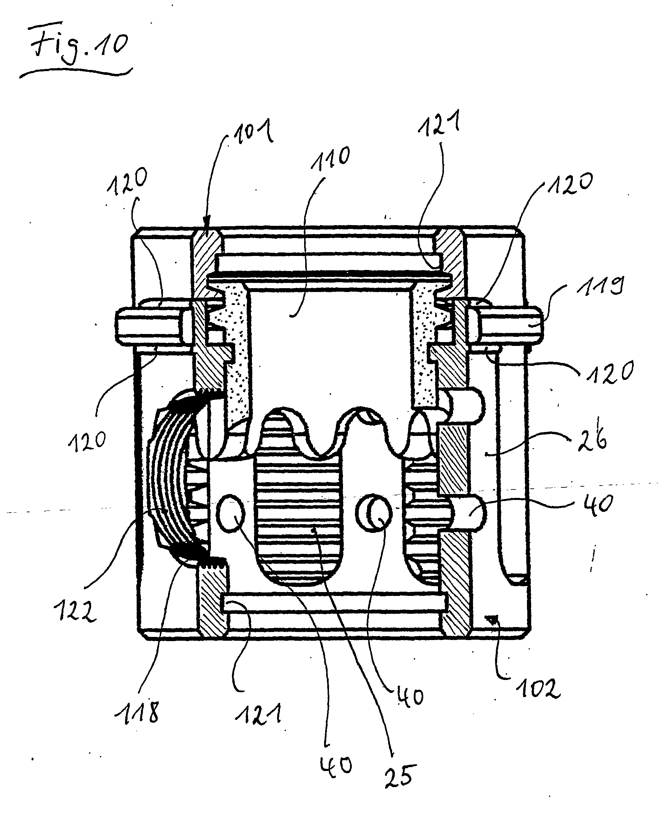

Zur Vergrößerung der Stützfläche der Hülsenteile 101, 102 sind an deren voneinander wegweisenden Stirnseiten 27, 35 radial über den Umfang der Hülsenteile 101, 102 hinausstehende Stützplatten 36 angeordnet, die von einer zentralen Öffnung 37 durchsetzt sind. Die Stützplatten 36 sind vorzugsweise lösbar fixiert. Diesem Zwecke dient eine unterseits an der Stützplatte 36 angeformte, die Öffnung 37 umgrenzende Schürze 38. Diese kann z.B. ein Außengewinde tragen, mit der die Stützplatte 36 in das erste Hülsenteil 101 einschraubbar ist. Bei der Ausführung nach Fig. 7, 7A ist jedoch eine Schnappverbindung vorgesehen. Dazu ist in einem Hülsenteil 101, 102 innenseitig eine Ringnut 121 (Fig. 10) vorhanden, in die an der Außenseite der Schürze 38 angeformte Rastvorsprünge 54 eingreifen. Die Schürze 38 ist durch stirnseitige Ausnehmungen 55 in vier Umfangsabschnitte 56 unterteilt. Deren die Rastvorsprünge tragenden Enden werden beim Einsetzen einer Stützplatte 36 in ein Hülsenteil 101, 102 elastisch radial nach innen abgelenkt, gehen aber infolge elastischer Rückstellkräfte wieder in ihre Ausgangslage zurück, wenn die Rastvorsprünge 54 in die Ringnut 121 einschnappen. Die dem Implantat abgewandte Seite der Stützplatte 36 trägt mehrere Vorsprünge in Form eines Zackenringes 39, der die Öffnung 37 konzentrisch umgibt. Der Zackenring 39 dient zum Festkrallen des Implantats an einem Wirbelkörper.

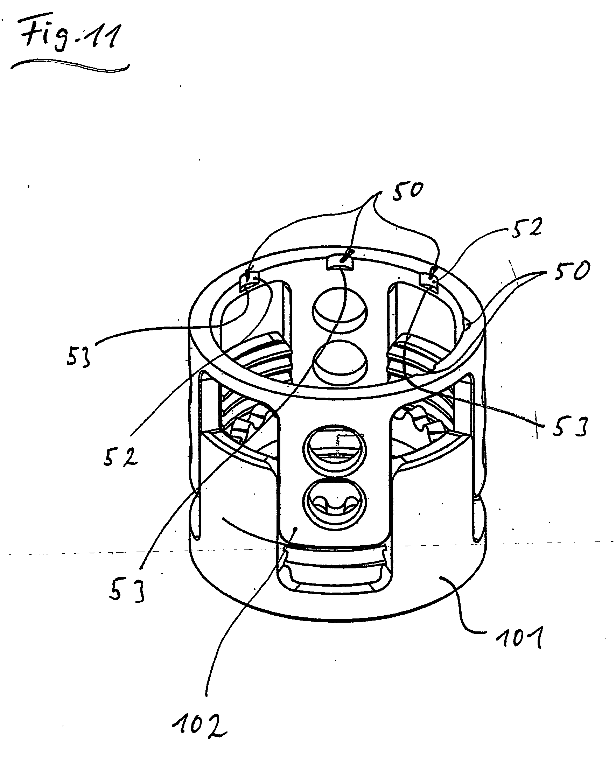

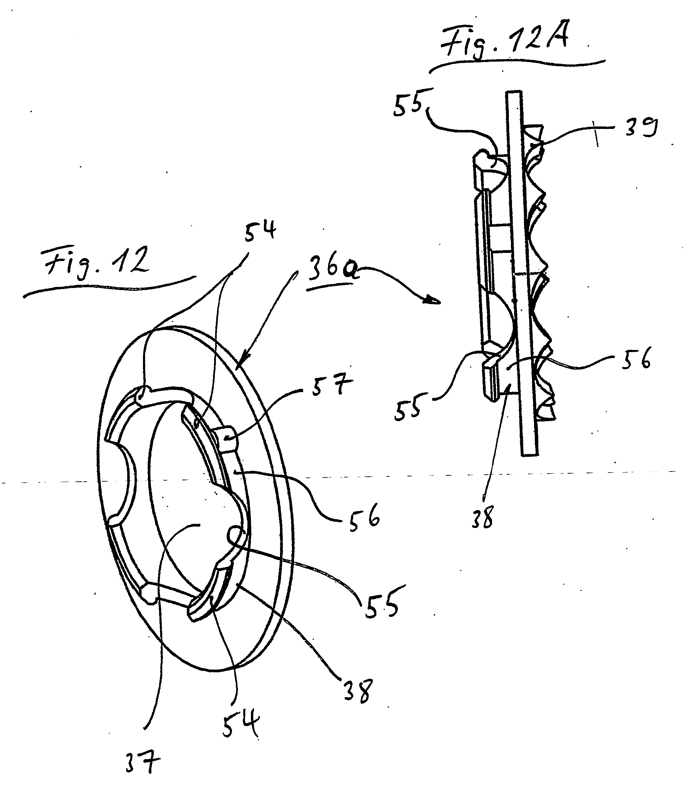

In Fig. 11 und 12 ist ein Ausführungsbeispiel gezeigt, bei dem an wenigstens einer Stirnseite eines Hülsenteils 101, 102 eine Stützplatte 36a drehfest fixiert ist. Dazu sind in den Stirnseiten 27, 35 der Hülsenteile 101,102, Ausnehmungen 50 vorhanden, die über den Umfang eines Hülsenteils 101, 102 z.B. gleichmäßig verteilt sind. Die Ausnehmungen 50 erstrecken sich in Axialrichtung und öffnen sich in die Innenseiten 51 der Hülsenteile. Die Ausnehmungen 55 sind von einer gekrümmten Innenwand 52 und einer Radialwand 53 begrenzt. Im Montagezustand greifen in die Ausnehmungen komplementär ausgebildete Vorsprünge 57 ein, welche an der Außenseite der Schürze 38 bzw. der Umfangsabschnitte 56 angeformt sind.

Im Hülsenteil 102 bzw. in dessen Umfangsabschnitten 26 sind zwei Öffnungen 40 vorhanden, über die Knochen- oder Knochenersatzmaterial in den Innenraum des Implantats einfüllbar ist. Es ist natürlich auch eine andere Anzahl von Öffnungen denkbar. Solche Öffnungen können auch im Hülsenteil 101 vorhanden sein. Wenigstens eine dieser Öffnungen bildet eine Zugangsöffnung 41 für ein im folgenden noch näher beschriebenes Handhabungswerkzeug. Der Zahnring 117 ragt in den Querschnitt der Zugangsöffnung 41 hinein.

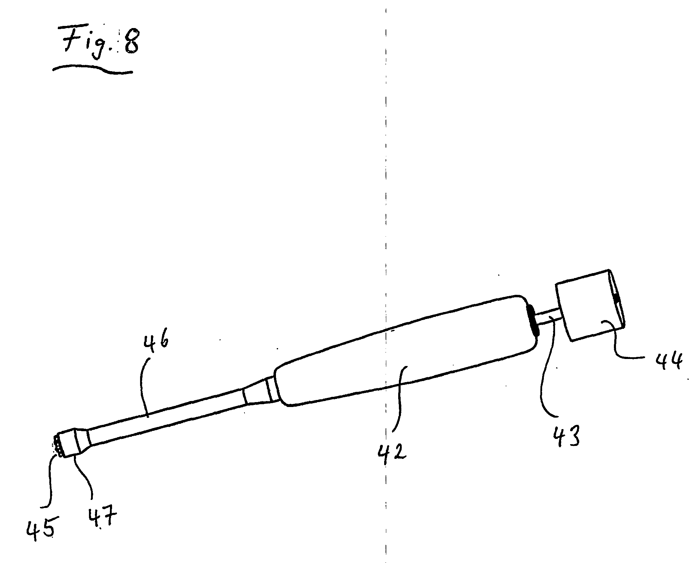

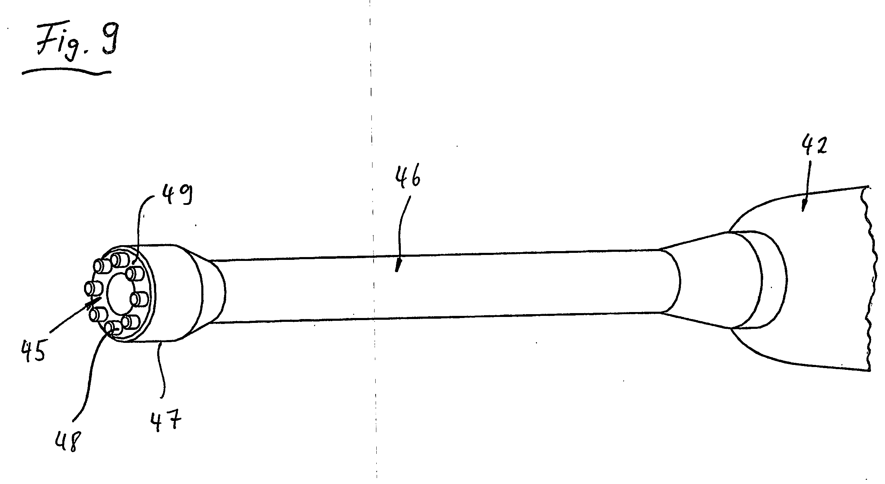

In Fig. 8 und 9 ist ein Handhabungswerkzeug gezeigt, das einen langgestreckten Handgriff 42, eine diesen zentral in Längsrichtung durchsetzende Welle 43, einen am einen Ende der Welle fixierten Drehgriff 44 und ein am anderen Ende der Welle fixiertes Zahnrad 45 als Haupt-Bauteile umfasst. Der sich zwischen dem Handgriff 42 und dem Zahnrad 45 erstreckende Abschnitt der Welle 43 verläuft innerhalb eines Hüllrohrs 46, das mit seinem, dem Zahnrad 45 abgewandten Ende am Handgriff 42 fixiert ist. Das Freiende 47 des Hüllrohrs 46 ist radial aufgeweitet. Die Zähne des Zahnrades 45 sind Bolzen 48, die in Längsrichtung der Welle 43 von der Vorderseite einer an dieser fixierten Scheibe 49 vorstehen. Zum Antrieb der Mutter 10 bzw. 110 wird das Handhabungswerkzeug mit seinem Freiende 47 in die Zugangsöffnung 18 bzw. 118 eingesteckt. Dabei gelangen die Bolzen 48 mit dem Zahnring 17 bzw. 117 in Eingriff. Diese Art der Verzahnung ist relativ robust und arbeitet auch dann noch zuverlässig und blockierungsfrei, wenn Material in den Verzahnungsbereich gelangt. Die Fixierung des Freiendes 47 in der Zugangsöffnung 1, 118 kann durch einen bloßen Reibschluss erfolgen. Es ist daher denkbar, dass das Freiende 47 mit einem Außengewinde in ein Innengewinde (Bezugszeichen 122 in Fig. 10) der Zugangsöffnung 18, 118 eingeschraubt wird.

Fig. 10 zeigt ein Ausführungsbeispiel mit einem Verstärkungsring 119. Dieser ist am oberen Ende des Umfangsabschnittes 26 des zweiten Hülsenteils 102 angeordnet. Von der Außenumfangsfläche der Umfangsabschnitte 26 stehen in Axialrichtung beabstandete Rippen 120 vor, die den Verstärkungsring 119 zwischen sich aufnehmen und in Axialrichtung fixieren. Der Verstärkungsring ermöglicht es, die Wandstärke der Umfangsabschnitte 26 des zweiten Hülsenteils 102 und auch die Umfangsabschnitte 25 des ersten Hülsenteils 101 zu reduzieren, ohne dass dabei die Gefahr besteht, dass die Umfangsabschnitte 25, 26 bei Druckbelastung radial nach außen aufgebogen werden.

Bei dem Ausführungsbeispiel nach Fig. 10 sind jeweils im oberen bzw. unteren Endbereich der Hülsenteile 101, 102 Nuten 121 in die entsprechenden Innenumfangsflächen eingearbeitet. In die Nuten 121 lässt sich eine Stützplatte 36 nach Art einer Schnappverbindung fixieren. Nuten 121 können auch bei den weiter oben beschriebenen Ausführungsbeispielen vorhanden sein.

Die Zugangsöffnung 118 weist ein Innengewinde 122 auf, in das ein Handhabungswerkzeug einschraubbar ist. Das Ausführungsbeispiel nach Fig. 10 zeigt darüber hinaus, dass die Zugangsöffnung 118 einen größeren Durchmesser aufweisen kann, als die übrigen Öffnungen 40 in den Umfangsabschnitten 25, 26.

### Bezugszeichenliste

- 1: Hülsenteil
- 2: Hülsenteil
- 3: Mutter
- 4: Mittellängsachse
- 5: Stirnseite
- 6: Stirnseite
- 7: Flansch
- 8: Oberseite
- 9: Flansch
- 10: Mutter
- 12: Flansch
- 13: Außengewinde
- 14: Innengewinde
- 14': Innengewindeabschnitt
- 15: Bereich
- 16: Bereich
- 17: Zahnring
- 18: Zugangsöffnung
- 22: Stirnseite
- 23: Stirnseite
- 24: Fenster
- 25: Umfangsabschnitt
- 26: Umfangsabschnitt
- 27: Stirnseite
- 28: Ringnut
- 29: Längsabschnitt
- 30: Längsabschnitt
- 33: Ringnut
- 34: Vorsprung
- 35: Stirnseite
- 36: Stützplatte
- 36a: Stützplatte
- 37: Öffnung
- 38: Schürze
- 39: Zackenring
- 40: Öffnung
- 41: Zugangsöffnung
- 42: Handgriff
- 43: Welle
- 44: Drehgriff
- 45: Zahnrad
- 46: Hüllrohr
- 47: Freiende
- 48: Bolzen
- 49: Scheibe
- 50: Ausnehmung
- 51: Innenseite
- 52: Innenwand
- 53: Radialwand
- 101: Hülsenteil
- 102: Hülsenteil
- 110: Mutter
- 113: Außengewinde
- 114: Innengewinde
- 117: Zahnring
- 118: Zugangsöffnung
- 119: Verstärkungsring
- 120: Rippe
- 121: Nut
- 122: Innengewinde

## Patentansprüche

1. Höhenverstellbares Implantat zum Einsetzen zwischen Wirbelkörpern, mit folgender Ausgestaltung:
- es weist ein erstes und ein zweites Hülsenteil (101, 102) auf,
- die Hülsenteile (101, 102) sind bei koaxialer Ausrichtung drehfest und axial beweglich miteinander verbunden,
- die Hülsenteile (101, 102) weisen axial verlaufende, in ihren einander zugewandten Enden ausmündende Fenster (24) auf wobei die zwischen zwei benachbarten Fenstern (24) eines Hülsenteils (101, 102) angeordneten Umfangsabschnitte (25, 26) in den Fenstern (24) des jeweils anderen Hülsenteils (101, 102) axial verschiebbar einliegen,
- **dadurch gekennzeichnet, dass**
- das erste Hülsenteil (101) ein Innengewinde (114) trägt,
- eine Mutter (110) in dem von den Hülsenteilen (101, 102)umgrenzten Innenraum koaxial angeordnet sowie drehbar und axialfest am zweiten Hülsenteil (102) fixierte ist,
- die Mutter (110) ein Außengewinde (113) trägt, das in das Innengewinde (114) des ersten Hülsenteils (101) eingreift,
- an der Mutter (110) ein koaxial zur Mittellängsachse (4) der Hülsenteile (101, 102) verlaufender Zahnring (117) angeordnet ist sowie,
- das zweite Hülsenteil (102) im Bereich des Zahnrings (117) von einer Zugangsöffnung (118) radial durchsetzt ist.

2. Implantat nach Anspruch 1,
**dadurch gekennzeichnet,**
**dass** der Zahnring (117) nach Art des Kronenrades eines Kronenradgetriebes ausgebildet ist.

3. Implantat nach Anspruch 1 oder 2,
**dadurch gekennzeichnet,**
**dass** die Mutter (110) so am zweiten Hülsenteil (102) fixiert ist, dass sich ihr Außengewinde (113) - in Axialrichtung gesehen - außerhalb des zweiten Hülsenteils (102) befindet.

4. Implantat nach Anspruch 3,
**dadurch gekennzeichnet,**
**dass** der Zahnring (117) auf einer vom Außengewinde (113) wegweisenden Fläche der Mutter (110) angeordnet ist.

5. Implantat nach Anspruch 3 oder 4,
**dadurch gekennzeichnet,**
**dass** an den Innenseiten der Umfangsabschnitte (26) des zweiten Hülsenteils (102) jeweils ein radial nach innen vorstehender Vorsprung (34) angeordnet ist, der in eine Ringnut (33) in der Außenfläche der Mutter (110) eingreift.

6. Implantat nach Anspruch 5,
**dadurch gekennzeichnet,**
**dass** die Vorsprünge (34) nahe dem Freiende der Umfangsabschnitte (26) angeordnet sind.

7. Implantat nach einem der Ansprüche 1 bis 6,
**dadurch gekennzeichnet,**
**dass** an der zur Anlage an einem Wirbelkörper vorgesehenen Stirnseite eines Hülsenteils (101, 102) eine Stützplatte (36) fixiert ist.

8. Implantat nach Anspruch 7,
**gekennzeichnet durch**
eine lösbare Fixierung der Stützplatte (36).

9. Implantat nach Anspruch 7 oder 8,
**dadurch gekennzeichnet,**
**dass** eine Stützplatte (36a) drehfest an einem Hülsenteil (101, 102) fixiert ist.

10. Implantat nach Anspruch 9,
**dadurch gekennzeichnet,**
**dass** in einer Stirnseite (27, 35) eines Hülsenteils (101, 102) mehrere in Umfangsrichtung beabstandete, sich in die Innenseite des Hülsenteils (101, 102) öffnende Ausnehmungen vorhanden sind, in die komplementär geformte Vorsprünge (57) an der Stützplatte (36a) eingreifen.

## Claims

1. Height-adjustable implant for insertion between vertebral bodies, the implant having the following design:
- it has a first and a second sleeve part (101, 102),
- the sleeve parts (101, 102) are oriented coaxially and connected to one another in a rotationally fixed and axially displaceable manner,
- the sleeve parts (101, 102) have axially extending windows (24) which open out in their mutually facing ends, and the circumferential sections (25, 26) arranged between two adjacent windows (24) of a sleeve part (101, 102) fit axially displaceably into the windows (24) of the respective other sleeve part (101, 102),
**characterized in that**
- the first sleeve part (101) has an internal thread (114),
- a nut (110) is arranged coaxially in the inner space delimited by the sleeve parts (101, 102) and is secured on the second sleeve part (102) in a rotatable but axially fixed manner,
- the nut (110) has an external thread (113) which engages in the internal thread (114) of the first sleeve part (101),
- a toothed ring (117), which is coaxial with respect to the central longitudinal axis (4) of the sleeve parts (101, 102), is arranged on the nut (110), and
- the second sleeve part (102) has an access opening (118) extending radially through it in the area of the toothed ring (117).

2. Implant according to Claim 1, **characterized in that** the toothed ring (117) is designed in the manner of the crown wheel of a contrate gear.

3. Implant according to Claim 1 or 2, **characterized in that** the nut (110) is secured on the second sleeve part (102) in such a way that its external thread (113), seen in the axial direction, is located outside the second sleeve part (102).

4. Implant according to Claim 3, **characterized in that** the toothed ring (117) is arranged on a surface of the nut (110) facing away from the external thread (113).

5. Implant according to Claim 3 or 4, **characterized in that,** on the inside faces of each of the circumferential sections (26) of the second sleeve part (102), a radially inwardly extending projection (34) is arranged which engages in an annular groove (33) in the outer surface of the nut (110).

6. Implant according to Claim 5, **characterized in that** the projections (34) are arranged near the free end of the circumferential sections (26).

7. Implant according to one of Claims 1 to 6, **characterized in that** a support plate (36) is secured on that end face of a sleeve part (101, 102) intended to bear on a vertebral body.

8. Implant according to Claim 7, **characterized by** the support plate (36) being secured in a releasable manner.

9. Implant according to Claim 7 or 8, **characterized in that** a support plate (36a) is secured on a sleeve part (101, 102) in a rotationally fixed manner.

10. Implant according to Claim 9, **characterized in that** several recesses, spaced apart in the circumferential direction and opening into the inside face of the sleeve part (101, 102), are present in an end face (27, 35) of a sleeve part (101, 102), and projections (57) of complementary shape arranged on the support plate (36a) engage in the recesses.

## Revendications

1. Implant réglage en hauteur à mettre entre des corps vertébraux et ayant la configuration suivante :
- il a une première et une deuxième parties (101, 102) formant douille,
- les parties (101, 102) formant douille sont assemblées entre elles sans possibilité de tourner dans la direction coaxiale et en étant mobiles axialement,
- les parties (101, 102) formant douille ont des fenêtres (24) s'étendant axialement et débouchant à leurs extrémités tournées l'une vers l'autre, les sections (25, 26) périphériques, disposées entre deux fenêtres (24) voisines d'une partie (101, 102) formant douille pouvant être mises en coulissant axialement dans les fenêtres (24) de respectivement l'autre partie (101, 102) formant douille, **caractérisé en ce que**
la première partie (101) formant douille porte un taraudage (114),
- un écrou (110) est disposé coaxialement dans l'espace intérieur délimité par les parties (101, 102) formant douille et est immobilisé de manière à pouvoir tourner et en étant fixe axialement sur la deuxième partie (102) formant douille,
- l'écrou (110) porte un filetage (113) qui engrène dans le taraudage (114) de la première partie (101) formant douille,
- sur l'écrou (110) est disposé un anneau (117) denté s'étendant coaxialement à l'axe (4) longitudinal médian des parties (101, 102) formant douille, ainsi que
- la deuxième partie (102) formant douille est traversée radialement par une ouverture (118) d'accès dans la zone de l'anneau (117) denté.

2. Implant suivant la revendication 1,
**caractérisé en ce que**
l'anneau (117) denté est constitué à la façon d'une roue de couronne d'un engrenage à roue de couronne.

3. Implant suivant la revendication 1 ou 2,
**caractérisé en ce que**
l'écrou (110) est immobilisé sur la deuxième partie (102) formant douille de façon à ce que son filetage (113) se trouve, tel que vu dans la direction axiale, à l'extérieur de la deuxième partie (102) formant douille.

4. Implant suivant la revendication 3,
**caractérisé en ce que**
l'anneau (117) denté est disposé sur une surface de l'écrou (110) s'éloignant du filetage (113) extérieur.

5. Implant suivant la revendication 3 ou 4,
**caractérisé en ce que**
sur les côtés intérieurs des sections (26) de pourtour de la deuxième partie (102) formant douille sont disposées respectivement des saillies (34) en saillie radialement vers l'intérieur et pénétrant dans des rainures (33) annulaires de la surface extérieure de l'écrou (110).

6. Implant suivant la revendication 5,
**caractérisé en ce que**
les saillies (34) sont disposées près de l'extrémité libre des sections (26) de pourtour.

7. Implant suivant l'une des revendications 1 à 6,
**caractérisé en ce qu'**une plaque (36) d'appui est immobilisée sur le côté frontal d'une partie (101, 102) formant douille, côté prévu pour s'appliquer à un corps vertébral.

8. Implant suivant la revendication 7, **caractérisé par** une immobilisation amovible de la plaque (36) d'appui.

9. Implant suivant la revendication 7 ou 8, **caractérisé en ce qu'**une plaque (36) d'appui est immobilisée sans pouvoir tourner sur une partie (101, 102) formant douille.

10. Implant suivant la revendication 9, **caractérisé en ce que** dans un côté (27, 35) frontal d'une partie (101, 102) formant douille sont prévues plusieurs ouvertures qui sont à distance dans la direction du pourtour qui s'ouvrent dans le côté intérieur de la partie (101, 102) formant douille et dans lesquelles les saillies (57) formées de manière complémentaire pénètrent sur la plaque (36a) d'appui.
